# EUROPEAN PATENT SPECIFICATION

(11) **EP 3 860 341 B1**
(45) Date of publication and mention of the grant of the patent: **02.12.2026**
(21) Application number: 19791103.5
(22) Date of filing: 03.10.2019
(51) Int. Cl.: A01N 1/144, A01N 1/146, C12N 5/00

(54) **SYSTEM FOR FREEZING, STORAGE, TRANSPORT, AND THAWING OF BIOPHARMACEUTICAL PRODUCT AND PALLET FOR USE IN SUCH A SYSTEM**
SYSTEM ZUM GEFRIEREN, LAGERN, TRANSPORTIEREN UND AUFTAUEN VON BIOPHARMAZEUTISCHEN PRODUKTEN SOWIE PALLETTE ZUR VERWENDING IN EINEM SOLCHEN SYSTEM
SYSTÈME DE CONGÉLATION, DE STOCKAGE, DE TRANSPORT ET DE DÉCONGÉLATION DE PRODUITS BIOPHARMACEUTIQUES ET PALETTE DESTINÉE À ÊTRE UTILISÉE DANS UN TEL SYSTÈME

(30) Priority: 05.10.2018 US 201816153172
(43) Date of publication of application: 11.08.2021
(73) Proprietor: Sartorius Stedim North America Inc., Bohemia, NY 11716 (US)
(72) Inventor: CUTTING, Jonathan, East Setauket, NY 11733 (US); SANCHEZ, Marc, Cambridge, MA 02141 (US)
(74) Representative: Novagraaf International SA
(86) International application number: PCT/US2019/054554
(87) International publication number: WO 2020/072810

(56) References cited:
- EP-A2- 1 134 000
- WO-A2-2012/135216
- US-A- 3 261 394
- US-A- 5 361 828
- US-A1- 2013 111 931
- US-A1- 2018 125 754

## Description

### FIELD OF THE INVENTION

The present disclosure relates to a freezing, storage, transport, and thawing system for disposable single-use containers containing biopharmaceutical products. More particularly, the system can be applied to disposable, single-use containers containing biopharmaceutical products.

### BACKGROUND OF THE DISCLOSURE

There is a need to transport containers (such as bags or semi-rigid containers) containing biopharmaceutical products from one facility to another facility. One efficient way to keep the products safe and sterile is to ship the products in a frozen state. At a first facility, the containers containing biopharmaceutical products are frozen, and then shipping is carried out, and at a second facility, the containers containing biopharmaceutical products are thawed. Since the containers contain expensive biopharmaceutical products, they must be protected from mechanical stresses and they are therefore enclosed in a casing or likewise packaging during the whole process of freezing, storage, transport, and thawing. However, the presence of the casing slows down thermal exchanges, and this extends the time required to freeze and thaw the bags. Another issue is the segregation that may occur inside the bag during phase change. Also, it is important to keep homogeneity of the biopharmaceutical product during temperature change.

High volume of biopharmaceutical products needs to be processed and known solutions exhibit poor productivity ratings.

Under this perspective, there is a need to propose new solutions to reduce the freezing and thawing time. Promoting homogeneity and preventing segregation is another target to meet.

WO 2012/135216 A2 discloses a system for freezing, storing, transporting, and thawing a biopharmaceutical product.

### SUMMARY OF THE DISCLOSURE

According to one aspect of the present invention, there is disclosed a system for freezing and thawing biopharmaceutical as defined in claim 1. The system comprises a stationary equipment and one or more movable pallet(s), each pallet being configured to carry one or more container unit(s), each containing biopharmaceutical product,
the stationary equipment comprising a gas circulating unit configured to cause a circulation of cold gas and/or warm gas, from a pull inlet toward a blow outlet,
wherein there is provided at least a first side plenum having one or more gas conveying channel(s) therein, configured to be fluidly coupled to the blow outlet of the gas circulating unit,
the first side plenum having a plurality of outlet ports directed toward an interior volume of the pallet, wherein the gas circulates substantially in a horizontal direction in the interior volume of the pallet.

Thanks to these dispositions, the gas circulation is precisely controlled, in terms of flow and path with regard to the containers to be frozen or thawed. The thermal exchanges between the biopharmaceutical product contained in the container(s) and the gas flow running along the top and bottom sides of the containers can be mastered, and process efficiency can be improved.

It should be understood by '*gas*' either simply air or a specific gas combination.

The term *'plenum'* shall be understood as synonymous to a manifold, i.e. a part having one or more inner volume(s) or recess(es) that are able to guide a flow of fluid (liquid or gas) from a plurality of inlets (converging flow) to a single outlet, or from a single inlet to to a plurality of outlets (diverging flow). Such 'plenum' may be formed in a single integral piece or in several assembled pieces. Such 'plenum' may have a mechanical supportive function or may be deprived of any mechanical supportive function.

Here, the biopharmaceutical product is typically a biopharmaceutical fluid, for example a fluid derived from a culture medium, a cell culture, a buffer solution, an artificial nutrition liquid, a blood fraction, a blood derived component or a pharmaceutical fluid or, more broadly, a fluid specifically designed to be used in the medical field The present disclosure also concerns handling of intermediate products that must be moved during manufacturing.

Here, the container unit can be construed as a container alone stricto sensu or as an assembly made of a container and associated protection means, as it will be discussed later. According to one option, there may be further provided a second side plenum having a plurality of inlet ports, wherein the outlet ports of the first side plenum are adapted to blow gas while the inlet ports of the second side plenum are adapted to pull gas and return the gas to the gas circulating unit, where the inlet ports are arranged respectively opposite the outlet ports in the horizontal direction. The second side plenum is configured to be fluidly coupled to the pull inlet of the gas circulating unit. Advantageously, a closed loop circuit is provided for the gas circulation, which improves the efficiency and controllability. This also improves the horizontality of the gas flow.

According to one option, the container units may be formed as bag assemblies, each bag assembly comprising a single-use flexible bag and a protective packaging, each single-use flexible bag containing biopharmaceutical product (BP). The protective packaging can be any type of casing, shell or frame substantially surrounding at least the border of the bag. It is therefore possible to handle flexible bags conveniently and safely.

The term *'pouch'* can also be used instead of *'bag'.* 'Vessel' can also be sometimes used instead or 'bag' or 'pouch'.

The flexible bag (or pouch) is generally designed for a single use and is adapted to contain a biopharmaceutical fluid volume which is here comprised between 5 liters and 100 liters, in some exemplary uses it is comprised between 10 liters and 30 liters. Advantageously this kind of bag, when empty before use, occupies a very small space, it can be flat, folded or even rolled up. Also, in most cases, the weight of the container unit allows a manual handling by qualified personnel without hauling assistance.

According to one advantageous aspect, the container units, respectively the single-use flexible bags, extend substantially in the horizontal direction. Whereby, the container units (resp. the bag assemblies) can be installed in a stable position (i.e. self-standing position), either on supports provided on the pallet or on a stack configuration (stacked on top of one another). This is consistent with horizontal gas flow and this maximizes the thermal transfer surface/area.

According to the invention, the system further comprises a shaker device. Such shaker device can include reciprocating, orbital, or other periodic movement for causing the mixing the content of each container unit. For example, reciprocating movement is imparted to the whole pallet, which improves the homogeneity of the biopharmaceutical product in each container unit (resp. flexible bag). Reciprocating movement can also prevent segregation if such function is required. Shaker can engage with the the base of the pallet.

According to one option, there may be further provided turbulators that favor turbulent gas flow along top and bottom sides of the containers. Such turbulators can include baffles. This promotes turbulent flow (versus laminar flow) and serves to break down the boundary layer; this increases thermal exchange coefficient, thereby resulting in a better overall efficiency.

According to one possible choice, the side plenums are arranged on the pallet. Stated otherwise, the one or two side plenums are mounted on the pallet base, via an optional damping/cushioning layer interposed between the pallet base and the side plenums. Under this configuration, the stationary equipment can be made rather compact with no floor occupation. Each pallet has its own plenum(s) with a simple and easy-to-couple interface with the gas circulating unit.

According to another possible choice, the side plenums are arranged on the stationary equipment. This gives a reduced footprint for the pallet(s). This also decreases the capital expenditure since pallets are simpler.

According to one option, the container units may be stacked on top of one another. Whereby, use of a shelf supporting system can be avoided in the pallet.

According to one option, there may be provided local gas guiding walls at top and bottom sides of each container unit. Such local gas guiding walls provide precise guidance of air flow along the bags/containers. Such local gas guiding walls can substantially surround each bag (each container). In the case of bags and protective packaging, appropriate local gas guiding means can be provided in the packaging itself (e.g. in the casing).

According to one option, there may be provided side supports to support the container units. Thereby, several container units (resp. bag assemblies) can be installed independently from one another in a pallet. Having several container units in a pallet enables to process a large volume of product at the same time, with independence concerning handling (it is not necessary to take/handle the top unit first).

According to one option, there may be horizontal separator plates arranged between two neighboring container units to provide gas guidance. Such separator plates are preferably arranged at distance from the container units themselves allowing thereby gas passage. This improves air flow controllability, promotes homogeneous/balanced thermal flow.

According to one option, the stationary equipment (or in practice the gas circulating unit) may comprise a blower, and a heat exchanger. The blower imparts the gas circulation, preferably under controlled speed/flow and the heat exchanger can be for example any air chiller, using a cold source. One example can be a liquid nitrogen exchanger to cool down air, which proves to be an efficient solution to provide cold air. A second example can be a conventional refrigerant-to-air heat exchanger which utilizes the latent heat of an evaporating refrigerant to cool the air.

According to one option, the stationary equipment (or in practice the gas circulating unit) may comprise an electrical heater. This is an efficient solution to provide warm, thawing air/gas and provides good controllability.

According to one option, the gas (i.e air) flow adjacent to the bags/containers is flowing in the same direction at upper side and lower side of the bag. This provides uniform boundary conditions between top and bottom respective portions. Temperature gradient across the thickness of the bag is thereby minimized.

According to one option, there are provided an inlet conduit at the first side plenum and an outlet conduit at the second side plenum for coupling the one or two plenums to the gas circulating unit. Easy coupling/decoupling, preferably obtained by the final approach movement when the pallet is docked to the gas circulating unit.

According to one option, there may be provided a closing cover that can be installed to cover the pallet for avoiding ingress of foreign objects during transport. The cover may have additionally a thermal barrier function.

According to one option, the pallet has a base with grooves for the passage of forklift arms, and easy handling with such forklift.

According to one option, the system may further comprise a control unit and one or more sensors, for monitoring or for controlling the freezing/thawing process in a closed loop fashion.

According to one option, the pallet may exhibit a footprint of substantially 80 cm X 120 cm. This is "Euro pallet" standard, which is an affordable base and exhibits excellent availability.

According to one option, there is defined for the pallet a pallet density index PDI=BPV/PLV where BPV is the total volume of the biopharmaceutical product (BP) contained in the container units housed in the pallet, where PLV is the height of the pallet multiplied by the footprint of the pallet (i.e. the overall volume), and wherein **PDI** is comprised between 0.1 and 0.4, preferably in a range [0.15 to 0.3]. This proves to be an optimum compromise for good usage of volume versus thermal flow efficiency.

According to one option, the protective packaging can comprise a local gas inner guiding wall in contact with the flexible bag, whereby preventing any contact between the circulating gas and the outer surface of the flexible bag, and allowing thermal flow therebetween.

According to one option, the protective packaging can be designed to promote a good contact between the circulating gas and the outer surface of the flexible bag.

According to a second aspect of the present invention, there is provided a pallet as defined in claim 13, the pallet being configured to be used in a system for freezing and thawing biopharmaceutical product, the pallet being configured to carry one or more container units, each containing biopharmaceutical product,
the pallet comprising a first side plenum having one or more gas conveying channel(s) therein, configured to be fluidly coupled to a blow outlet of a gas circulating unit,
the first side plenum having a plurality of outlet ports directed toward an interior volume of the pallet, wherein the gas circulates substantially in a horizontal direction (XY) in the interior volume of the pallet. The pallet further comprises a shaker device.

According to one option, the pallet may further comprise a second side plenum having a plurality of inlet ports, the second side plenum being configured to be fluidly coupled to the pull inlet of the gas circulating unit, wherein the outlet ports of the first side plenum are adapted to blow gas while the inlet ports of the second side plenum are adapted to pull gas and return the gas towards the gas circulating unit, where the inlet ports are arranged opposite the outlet ports in the horizontal direction.

### BRIEF DESCRIPTION OF THE DRAWINGS

Other features and advantages of the invention appear from the following detailed description of one of its embodiments, given by way of non-limiting example, and with reference to the accompanying drawings, in which:
- Figure 1 illustrates a diagrammatic perspective view of a pallet according to a first embodiment.
- Figure 2 is an exemplary diagrammatic exploded view of a container unit received in the pallet shown at figure 1,
- Figure 3 is a diagrammatic front view of the pallet shown at figure 1,
- Figure 4 is an exemplary diagrammatic elevation side view of a plenum used in the pallet shown at figure 1,
- Figure 5 is a diagrammatic top view of the pallet shown at figure 1,
- Figure 6 is a diagrammatic section view of an exemplary container unit,
- Figure 7 is analogous to figure 3 and illustrates a second embodiment,
- Figure 8 and 9 show views of the complete system according to the second embodiment,
- Figure 10 illustrates an exemplary gas circulating unit,
- Figure 11 is another diagrammatic section view of an exemplary container unit,
- Figure 11A is a zoomed view showing turbulators,
- Figure 12 is exemplary block diagram of the control system,
- Figure 13 is analogous to Fig 7 and shows a variant embodiment,
- Figure 14 is a diagrammatic side view of the pallet in a docking position.

### DETAILED DESCRIPTION OF THE DISCLOSURE

In the figures, the same references denote identical or similar elements. For the sake of clarity, some elements may not be represented at scale.

In the context of the present disclosure, there are provided containers (such as bags or semi-rigid containers) containing biopharmaceutical product (denoted **BP)** which need to be shipped from one facility to another facility under the frozen form.

Here, the biopharmaceutical product is typically a biopharmaceutical fluid, for example a fluid derived from a biopharmaceutical process, like a culture medium, a cell culture, a buffer solution, a blood fraction, or any fluid specifically designed to be used in the medical field. The biopharmaceutical product at stake can also be any intermediate products that must be moved during manufacturing.

Therefore, at one place, there is a need to freeze such containers (or container units) containing biopharmaceutical product, and after transport, at another place, there is a need to thaw such containers containing biopharmaceutical product, such that the biopharmaceutical product can be used and further processed in the liquid form.

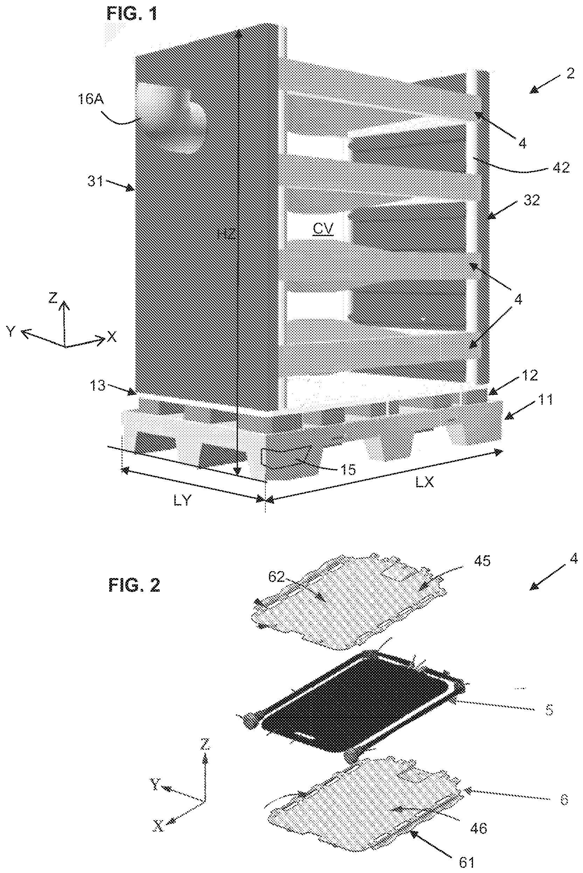

Freezing, storage, transport, and thawing are performed while the containers are arranged in a pallet **2.** As shown in figure 1, the pallet **2** comprises a base **11,** an optional cushioning/damping layer **12,** and optionally a support plate **13.** Additionally, there is provided a shaker device **15,** the latter being described later. The pallet 11 has a base with grooves **11G** for the passage of forklift arms, and easy handling with such forklift.

The damping layer **12** can be formed as spaced rubber pads. The support plate **13** can be formed as a thin metal plate, for example from stainless steel.

The pallet 2 exhibits a substantially standard footprint. LY can be 80 cm, LX can be 120 cm. Height HZ can be comprised between 1m and 2m, preferably between 1,2 m and 1,6 m

The pallet **2** carries one or more container units **4,** each containing biopharmaceutical product BP.

Various types of container unit are considered in the scope of the present disclosure. According to one example, the container unit may be formed as a bag assembly **4,** each bag assembly comprising a single-use flexible bag **5** and a protective packaging **6,** each single-use flexible bag containing biopharmaceutical product **BP.** The protective packaging can be any type of casing, shell or frame substantially surrounding at least the border of the bag.

In practice, the protective packaging **6** may be formed as two shells in halves **61,62,** namely a bottom shell and a top shell. One example of protecting packaging can be found in the published document US2018-125754 from the same applicant.

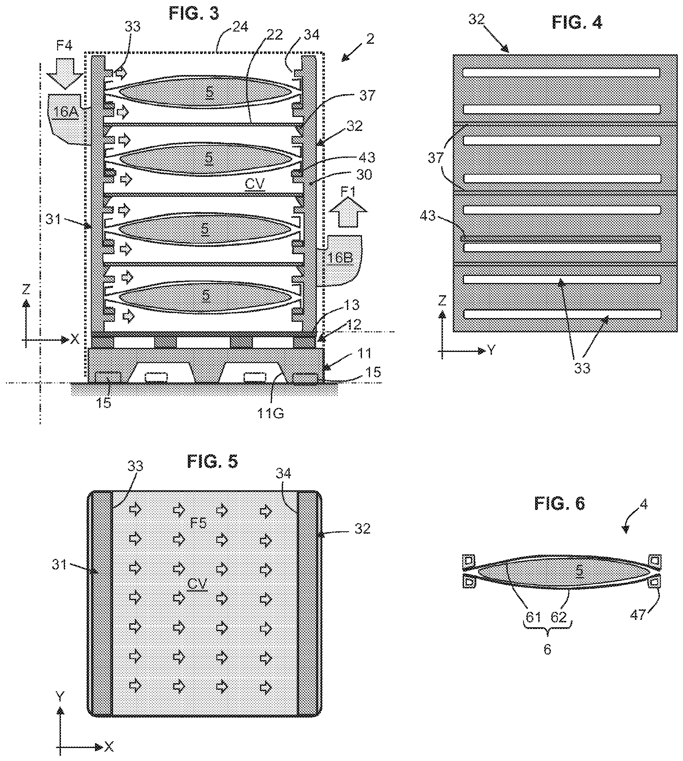

There can be provided a first level of packaging formed as plates as described in US2018-125754, such plates are designed to sandwich substantially the bag 5. Such plates are shown at the figures 2 and 6 by reference numerals **61** and **62.**

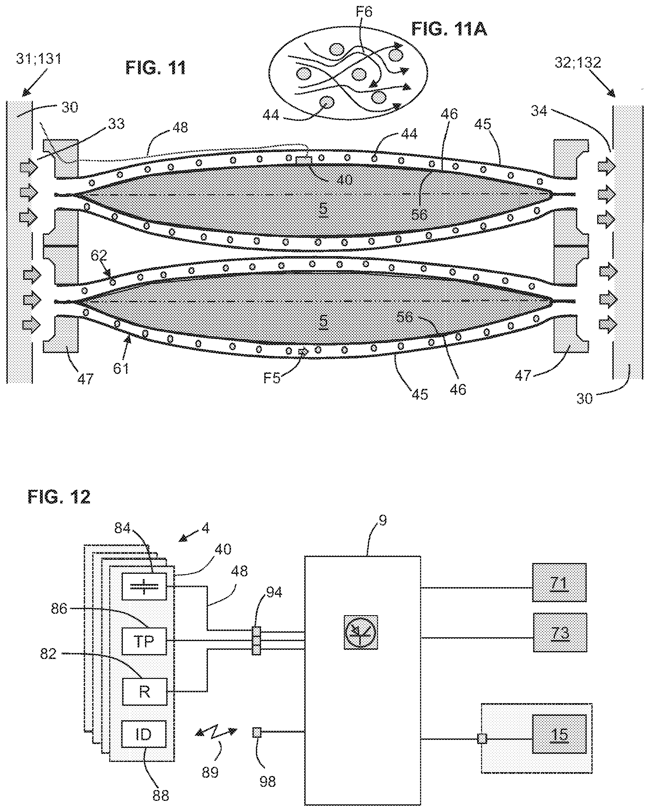

This first level of packaging can be complemented by a second level of packaging formed as frames **47** which sandwich the plates, as visible at figures 6 and 11. Such frames extend in a peripheral fashion and form strength members or armatures that provide overall rigidity and allow easy handling of the container units **4** in the whole process. Plates and frames can be made of PET or HPDE. More details on plates and frames can be found in US2018-125754.

In one embodiment, there may be provided a hose arrangement for filling and draining the bag, such hose arrangement begin encompassed between the borders of the plates.

Regarding the bags 5 themselves, one can use for example available products like the bag product range Flexboy^{®} from the applicant company Sartorius Stedim Biotech^{®}. Other single use flexible bags can also be used within the context of the present invention.

According to another example, the container unit is formed as a semi-rigid container directly containing the biopharmaceutical product BP. In this configuration, the container unit could be a single container alone, without additional protection means.

In any case, intrinsic protection means or additional protection means allows achievement of a good level of mechanical protection. It is therefore possible to handle flexible bags conveniently and safely.

Protection means also allows to place the container units safely in a horizontal position where they can be in a self-standing and stable state.

Spatial reference is given by horizontal directions **X** and **Y** and vertical direction **Z. X** denotes left- right direction from the forklift user standpoint (from where the pallet is handled with a forklift), whereas **Y** denotes front-back direction perpendicular to **X.** We note that docking movement is substantially along **Y** axis.

In a first embodiment, according to the present disclosure, there is provided at least a first side plenum **31** and a second side plenum **32.**

First side plenum **31** extends generally in a **YZ** plane. Second side plenum **32** extends generally in a **YZ** plane, but in a position opposite to the first plenum regarding the transverse axis **X.** First and second side plenums **31,32** exhibit generally a parallelepiped shape. In the shown example, they are generally symmetrical to one another regarding a median **YZ** plane.

Each side plenum can be manufactured from stainless steel or inert aluminium/tin alloy. Also high performance biocompatible plastics can be used. Also fiber loaded resin or polymer can be used.

Each of first and second side plenums **31,32** has one or more gas conveying channel(s) therein. According to one example, each of first and second side plenums **31,32** can be built as a hollow body. In one example, there is no partition into this hollow body and the overall recess formed by the interior of the hollow body constitutes a single gas conveying channel.

In other embodiments, there may be provided more specific recesses like pipes each forming a specific channel. Even, each of first and second side plenums **31,32** can be formed as a specific manifold.

Here the plenum has a hollow body with two main plates; the plenum hollow body has the plurality of inlet/outlet ports in one of the plate oriented toward the interior volume **CV** of the pallet and a connection opening for supply (resp discharge) in the other plate oriented toward the exterior of the pallet.

Gas conveying channel(s) of first side plenum **31** convey gas from an inlet duct **16A** to outlet ports **33** directed toward an interior volume **CV** of the pallet.

Gas conveying channels of second side plenum **32** convey gas from plurality of inlet ports **34** to an outlet duct **16B.**

In the illustrated example, each outlet port **33** is formed as slot open to the plenum hollow body; the slot extends in the Y direction, and extends substantially along the whole length of the plenum along Y. Instead, there can be provided a plurality through-holes, like nozzles.

At the right side, each inlet port **34** of the side plenum **32** is also formed as slot open to the plenum hollow body; the slot extends in the Y direction, and extends substantially along the whole length of the plenum along Y. There are preferably two slots per container units.

As apparent from the figures, from the forklift user standpoint, the first side plenum **31** is a left side plenum and the second side plenum **32** is a right side plenum. Further, there may be provided a cover **24,** delimiting the interior volume **CV** of the pallet on the top side, the front side and the backside. Stated otherwise, the interior volume **CV** is delimited on the left and right sides by the plenums **31,32,** on the bottom side by the pallet base **11** (or plate **13)** and on the remaining sides (top, front, bottom) by the specific cover **24.**

The cover **24** is removably installed on the pallet for transport steps. The cover **24** is removed when filling, draining or handling the container units 4. The cover **24** is removed for the freezing and thawing process. The cover 24 provides mechanical protection; it avoids inadvertent ingress of foreign objects in the pallet inner volume CV during transport. In some embodiments, the cover exhibits additionally a thermal barrier function.

As understood from figures 3, 4 and 5, the gas circulates substantially in a horizontal direction **XY** in the interior volume **CV** of the pallet, i.e. from outlet ports **33** of the first side plenum 31 to the horizontally corresponding inlet ports **34** of the second side plenum 32. This horizontal flow is denoted by arrow **F5** (fig 5).

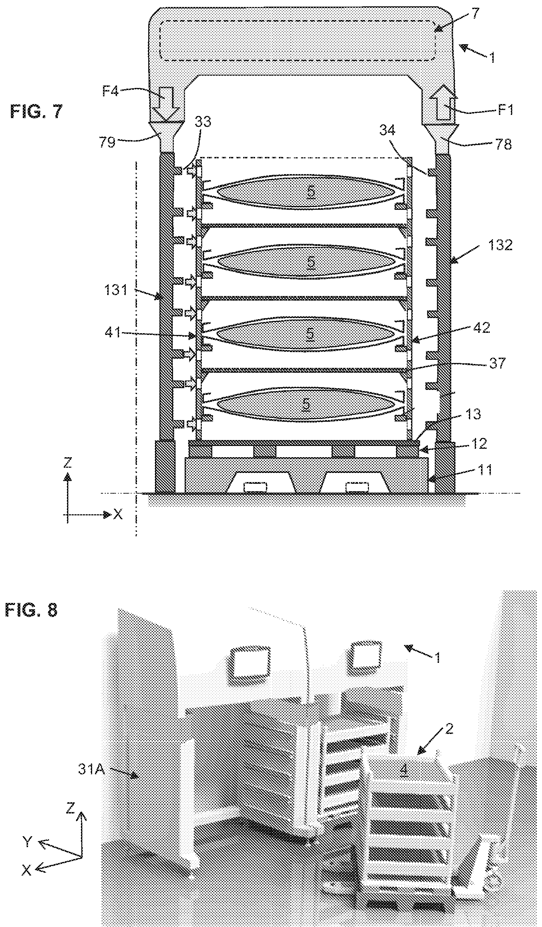

Regarding now the way container units 4 are mechanically supported within the pallet, there are considered various solutions. As depicted in figures 1, 3, 7, each container unit is supported on its left and right sides, either directly by the above mentioned plenums or by specific uprights **41,42** (cf Fig 7).

In other words the above mentioned plenums can comprise therein the upright function or can be deprived of such upright function, in which case the pallet **2** comprises additionally the above mentioned specific uprights **41,42.**

Each container unit **4** can be supported on side slides which extend along Y, or by simple shelf supports **43.** It is understood that said side slides or simple shelf supports **43** can be arranged directly on the interior side of the plenums or arranged on the specific uprights **41,42.**

As understood from figure 3, there may be provided additionally horizontal separator plates **22** arranged between two neighboring container units to provide gas guidance. The horizontal separator plates **22** can be arranged on side slides **37** or otherwise on simple shelf supports.

In the case where side slides (resp. simple shelf supports) are arranged directly on the interior side of the plenums, as depicted at figure **4****,** the interior side of the plenums exhibits an alternate succession of outlet ports **33,** support **43** for the container units, and side slides **37** for the separator plates **22.**

For each container unit **4,** there may be provided two outlet ports **33** on the left side plenum, namely one directed to the bottom of the container unit and one directed to the top of the container unit.

In a similar manner, when the second plenum is present, there may be provided two inlet ports **34** on the right side plenum 32, namely one facing the bottom of the container unit and one facing the top of the container unit.

Further, the disclosed system further includes a stationary equipment **1** to which the pallet(s) are to be docked and fluidly coupled.

The stationary equipment **1** comprises a gas circulating unit **7** (not shown at figure 1 but illustrated at figures 7 to 10). When it is under operation, the gas circulating unit causes a circulation of cold gas or warm gas (according to the respective need of freezing or thawing), from a pull/suck inlet **76** toward a blow outlet **77.**

The gas circulating unit comprises a blower **71,** and a heat exchanger **72.**

The gas circulating unit comprises an electrical heater **73.**

The gas circulating unit is power supplied from electrical network (mains). The pallets are brought in and out from a location situated below the gas circulating unit 7.

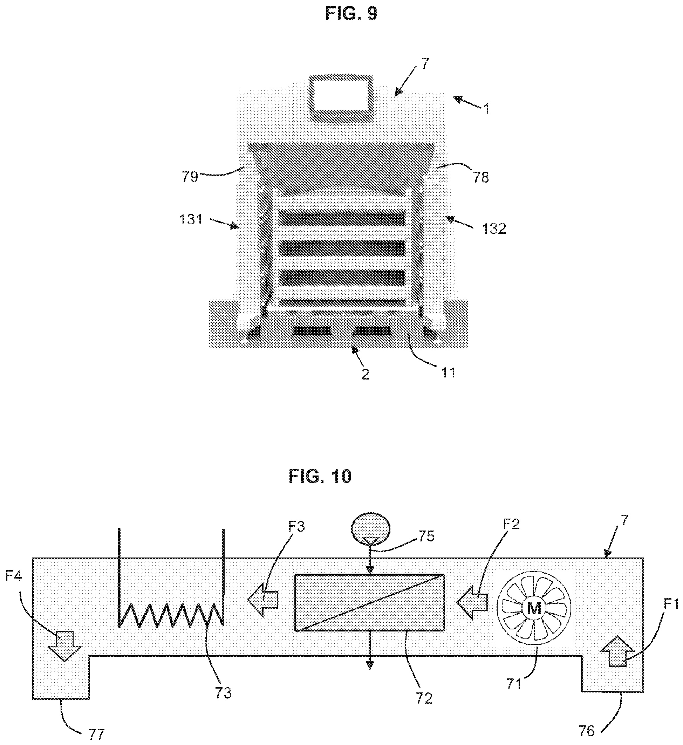

Regarding the following elements : blower **71,** heat exchanger **72,** electrical heater **73,** various arrangements are possible and the order of these elements from the pull inlet **76** toward to the blow outlet **77** can be different from the one which is illustrated at figure 10.

In the shown example, the heat exchanger **72** removes calorie from the air. In practice, it can be in evaporator. Any chilled fluid can be used in the heat exchanger **72** to cool air from **F2** to **F3** flow sections. Using liquid nitrogen is not excluded. There may be provided a circulation pump **75** to circulate a chilled fluid into the heat exchanger **72.**

Air/gas circulates in a channel along a flow shown by arrows F1,F2,F3,F4.

As illustrated in figures 7 to 9, according to a second embodiment, the plenums are not carried by the pallet, by the plenums are stationary. The plenums are arranged as part of the stationary equipment **1.** There is provided a first side plenum **131** standing on a socket and connected via a funneling means to the blow outlet **77** of the gas circulating unit 7.

In the illustrated example of figure 7, there is provided a second side plenum 132 standing on another socket and connected via a funneling means 78 to the pull/suck inlet **76** of the gas circulating unit 7.

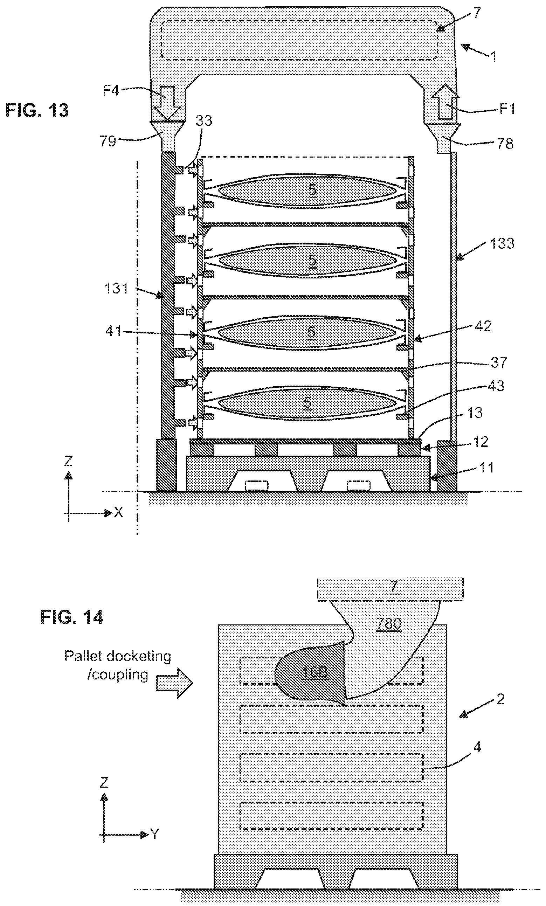

However, the person in the art can recognize from Fig 13 that the side plenum **132** is not strictly necessary. The return path from the interior volume CV to the pallet the gas circulating unit 7 can be managed through a simple side wall **133.**

The same can apply to the first embodiment where the second side plenum is not strictly necessary.

Gas conveying channel(s) of first side plenum **131** convey gas from an inlet conduit **79** to outlet ports **33** directed toward an interior volume **CV** of the pallet.

Gas conveying channels of second side plenum **132,** when present, convey gas from interior volume **CV** through the inlet ports **34** and toward the connecting conduit **78.**

There is provided a gap between the left upright **41** and the left plenum **31** and a gap between the right upright **42** and the right plenum **32.**

It should be noticed that the air flow adjacent to the bags is flowing in the same direction **F5** (from left to right at figures 3 and 5) at upper side and lower side of the bag.

There may be further provided local gas guiding walls **45** at top and bottom sides of each container unit, as illustrated at figure 11. Such local gas guiding walls may be formed in the protective packaging 6. In other words, the packaging 6 can envelop substantially a gas passage provided along the flexible bag.

Also, in one embodiment, each of the plates **61,62** of the protective packaging **6** can comprise an inner wall **46** and an outer wall **45** delimiting the passage for the gas flow.

According to one optional feature, the inner guiding walls **46** can be designed to prevent any contact between the circulating gas and the outer surface **56** of the flexible bag **5.** The inner guiding wall **46** is in contact with the outer surface **56** of the flexible bag thereby allowing conductive thermal flow therebetween to freeze or thaw the biopharmaceutical product **BP.** Accordingly, the inner guiding walls 46 exhibit low thermal resistance and good thermal conductivity.

According to an altemative feature, the inner guiding walls **46** can be designed to promote a good contact between the circulating gas and the outer surface **56** of the flexible bag 5. In this case, the inner guiding walls **46** can be designed as a grid with a plurality of large through holes.

There may be further provided turbulators **44,** which are devices designed to promote turbulent flow **F6** along top and bottom sides of the container units. Turbulent flow exhibits a better thermal exchange coefficient than laminar flow. There may be provided baffles or simply an array of rods **44** arranged transversally to the gas flow, as shown at Fig 11A.

Various solutions are envisioned for turbulators. The rods (or pins or studs) **44** extend between the inner walls **46** and the outer walls 45 delimiting the passage for the gas flow. The rods are arranged in an array and force the air to flow in a meandering path thereby increasing velocity, turbulence, and heat transfer coefficient.

Another solution is described in US5361828A wherein a staggered array of ramp surfaces arranged on inner walls **46** and outer walls **45.** Such ramp surfaces promote heat transfer by trailing vortices. This is optimized for flow in a single direction (here **F5).**

Generally speaking, any protruding or outstanding elements standing across the air passage tends to promote vortices and turbulent flow, which improves the heat transfer efficiency.

As apparent from figure 11, the container units may be piled up on one another avoiding use of a shelf supporting system in the pallet. Frame **47** has a generally flat bottom face and has a generally flat upper face, such that the bottom face of one container unit may bear in a stable manner on the upper face of another frame. Such stack configuration can be used both in the first embodiment (plenums on pallet) and in the second embodiment (stationary plenums).

Further, the system comprises a control unit **9** to control the freezing and thawing process. Further, the system comprises one or more sensors **82,84,86.** Such sensor(s) provide data which reflects the ongoing freezing process or thawing process.

There may be provided a thermocouple or NTC resistor sensor denoted **82,** responsive of a temperature sensed at bag contact.

There may be provided a capacitive sensor **84,** which is responsive to the liquid or solid consistence of the biopharmaceutical product, and notably responsive of a phase change of the biopharmaceutical product.

There may be provided a thermopile sensor **86** which is responsive to the liquid or solid consistence of the biopharmaceutical product, and notably responsive of a phase change of the biopharmaceutical product.

Either one or two or three of the above-mentioned sensors can be arranged in a sensing assembly **40.** The sensing assembly **40** may be coupled to control unit **9** via one or more wire(s) **48.**

The sensing assembly 40 may be arranged in a belly-button position, as shown at figure 11.

Further, the system may comprise means for identifying unitarily each container unit. As apparent from figure 12, each container unit may carry an identifier ID denoted **88,** like for example a RFID chip or a NFC chip. There may be provided a short range reader **98.** The short range reader **98** can be located in the pallet or outside the pallet, for example in the stationary equipment. Reading is made via wireless communication **89.**

In the components involved in the control system, some of them may be subjected to gamma irradiation for sterility purposes. In particular, the one or more sensors **82,84,86** (possibly as an assembly **40)** and the identifier ID **88** may be attached to the bag and form a connectable module subjected to gamma irradiation, whereas the rest of the system is not subject to gamma irradiation. A connector denoted **94** separates the gamma irradiated portion from the rest of the components involved in the control system.

The shaker device **15** can produce an orbital reciprocating movement in the XY plane. The shaker device **15** can produce a to-and-fro movement in the vertical direction Z.

There may be provided one or more shaker device(s) at pallet base 11.

For example, reciprocating movement is imparted to the whole pallet// Each bag assembly.

Fig 14 shows a convenient coupling via funnel, together with pallet final move in docking position along the Y direction.

Note : the term 'stationary' does not exclude an equipment that are mounted on rollers and that can be moved if required. But it is not moved for the treatment of pallets.

Regarding the overall arrangement within the pallet, we define for the pallet a pallet density index **PDI=BPV/PLV** where **BPV** is the total volume of the biopharmaceutical product **BP** contained in the container units housed in the pallet, where **PLV** is the height of the pallet multiplied by the footprint of the pallet (i.e. the overall volume).

The inventors have achieved a density rating with **PDI** comprised between 0.1 and 0.4, preferably in a range [0.15 to 0.3]. This proves to be an optimum compromise for good usage of volume versus thermal flow efficiency.

## Claims

1. A system for freezing, storing, transporting and thawing biopharmaceutical products, the system comprising a stationary equipment (1) and one or more movable pallets (2), each pallet being configured to carry one or more container units (4), each containing biopharmaceutical product (BP),
the stationary equipment comprising a gas circulation unit (7) configured to cause a circulation of cold gas and/or warm gas from a pull inlet (76) toward a blow outlet (77), wherein there is provided at least a first side plenum (31;131), having one or more gas conveying channel(s) therein, configured to be fluidly coupled to the blow outlet of the gas circulation unit,
the first side plenum (131) has a plurality of outlet ports (33) directed toward an interior volume (CV) of the pallet, wherein in use the gas circulates substantially in a horizontal direction (XY) in the interior volume of the pallet,
**characterized in that** the system further comprises a shaker device (15).

2. The system according to claim 1, wherein there is further provided a second side plenum (32;132) having a plurality of inlet ports (34), wherein the outlet ports (33) of the first side plenum are adapted to blow the gas while the inlet ports of the second plenum are adapted to pull gas and return the gas to the gas circulation unit (7), where the inlet ports are arranged opposite the outlet ports in the horizontal direction (XY).

3. The system according to any of the claims 1 to 2, wherein the system comprises one or more container units (4) configured to contain biopharmaceutical product (BP) and wherein the container units are bag assemblies, each bag assembly comprising a single-use flexible bag (5) and either a protective packaging frame or a protective packaging casing (6), each single-use flexible bag configured to contain a biopharmaceutical product (BP).

4. The system of claim 3, wherein in use of the system the single-use flexible bags extend substantially in the horizontal direction (XY).

5. The system according to any of the claims 1 to 4, wherein there are further provided turbulators (44) that favor turbulent gas flow along top and bottom sides of the container units.

6. The system according to any of the claims 1 to 5, wherein the side plenums (31,32) are arranged on the pallet.

7. The system according to any of the claims 1 to 5, wherein the side plenums (131,132) are arranged on the stationary equipment.

8. The system of any of the claims 1 to 7, wherein the system comprises two or more container units (4) configured to contain biopharmaceutical product (BP), wherein in use the two or more container units may be stacked on top of one another, and wherein there are provided local gas guiding walls (45) at the top and bottom sides of each container unit.

9. The system according to any of the claims 1 to 8, further comprising horizontal separator plates (22) arranged between two neighboring container units (4) to provide gas guidance.

10. The system according to any of the claims 1 to 9, wherein the stationary equipment (1) comprises a blower (71) and a heat exchanger (72), and/or wherein the stationary equipment (1) comprises an electric heater (73).

11. The system according to any of the claims 1 to 10, wherein the pallet exhibits a footprint of substantially 80 cm by 120 cm and wherein there is defined for the pallet a pallet density index PDI=BPV/PLV, where BPV is the total volume of the biopharmaceutical product (BP) contained in use in the container units and where PLV is the height of the pallet multiplied by the footprint of the pallet, i.e. the overall volume, and wherein PDI is comprised between 0.1 and 0.3.

12. The system according to claim 3 or claim 4, or any of the claims 5-11 if dependent on claim 3 or claim 4, wherein the protective packaging (6) comprises a local gas inner guiding wall (46) in contact with the flexible bag (5), thereby preventing any contact between the circulating gas and the outer surface (56) of the flexible bag (5), and allowing thermal flow therebetween.

13. A pallet (2) configured to be used in a system for freezing, storing, transporting and thawing biopharmaceutical product, the pallet being configured to carry one or more container units (4), each containing a biopharmaceutical product (BP),
wherein the pallet comprises a first side plenum (31) having one or more gas conveying channel(s) therein, configured to be fluidly coupled to a blow outlet of a gas circulating unit,
the first side plenum (31) has a plurality of outlet ports (33) directed toward an interior volume (CV) of the pallet, wherein in use gas circulates substantially in a horizontal direction (XY) in the interior volume of the pallet, the pallet further comprising a second side plenum (32) having a plurality of inlet ports (34), wherein the outlet ports (33) of the first side plenum are configured to blow gas while the inlet ports of the second side plenum are configured to pull gas and return the gas towards the gas circulation unit (7), where the inlet ports are arranged opposite the outlet ports in the horizontal direction (XY), wherein the side plenums (31, 32) further form uprights for supporting the container units (4),
**characterized in that**
the pallet further comprises a shaker device (15).

## Patentansprüche

1. System zum Einfrieren, Lagern, Transportieren und Auftauen von biopharmazeutischen Produkten, wobei das System eine stationäre Ausrüstung (1) und eine oder mehrere bewegliche Paletten (2) umfasst, wobei jede Palette dazu ausgebildet ist, eine oder mehrere Behältereinheiten (4) zu tragen, die jeweils ein biopharmazeutisches Produkt (BP) enthalten,
wobei die stationäre Ausrüstung eine Gaszirkulationseinheit (7) umfasst, die dazu ausgebildet ist, eine Zirkulation von kaltem Gas und/oder warmem Gas von einem Ansaugeinlass (76) hin zu einem Ausblasauslass (77) zu bewirken,
wobei mindestens ein erstes Seitenplenum (31 ; 131) vorgesehen ist, das ein oder mehrere Gasförderkanäle darin aufweist und dazu ausgebildet ist, fluidisch mit dem Ausblasauslass der Gaszirkulationseinheit gekoppelt zu werden,
wobei das erste Seitenplenum (131) eine Vielzahl von Auslassöffnungen (33) aufweist, die hin zu einem Innenvolumen (CV) der Palette gerichtet sind, wobei im Gebrauch das Gas im Wesentlichen in einer horizontalen Richtung (XY) in dem Innenvolumen der Palette zirkuliert,
**dadurch gekennzeichnet, dass** das System ferner eine Rütteleinrichtung (15) umfasst.

2. System nach Anspruch 1, wobei ferner ein zweites Seitenplenum (32 ; 132) vorgesehen ist, das eine Vielzahl von Einlassöffnungen (34) aufweist, wobei die Auslassöffnungen (33) des ersten Seitenplenums dazu ausgelegt sind, das Gas auszublasen, während die Einlassöffnungen des zweiten Plenums dazu ausgelegt sind, Gas anzusaugen und das Gas zu der Gaszirkulationseinheit (7) zurückzuführen, wobei die Einlassöffnungen in der horizontalen Richtung (XY) den Auslassöffnungen gegenüberliegend angeordnet sind.

3. System nach einem der Ansprüche 1 bis 2, wobei das System eine oder mehrere Behältereinheiten (4) umfasst, die dazu ausgebildet sind, ein biopharmazeutisches Produkt (BP) zu enthalten, und wobei die Behältereinheiten Beutelanordnungen sind, wobei jede Beutelanordnung einen flexiblen Einwegbeutel (5) und entweder einen Schutzverpackungsrahmen oder ein Schutzverpackungsgehäuse (6) umfasst, wobei jeder flexible Einwegbeutel dazu ausgebildet ist, ein biopharmazeutisches Produkt (BP) zu enthalten.

4. System nach Anspruch 3, wobei sich im Gebrauch des Systems die flexiblen Einwegbeutel im Wesentlichen in der horizontalen Richtung (XY) erstrecken.

5. System nach einem der Ansprüche 1 bis 4, wobei ferner Turbulatoren (44) vorgesehen sind, die einen turbulenten Gasstrom entlang der Ober- und Unterseiten der Behältereinheiten begünstigen.

6. System nach einem der Ansprüche 1 bis 5, wobei die Seitenplenen (31, 32) an der Palette angeordnet sind.

7. System nach einem der Ansprüche 1 bis 5, wobei die Seitenplenen (131, 132) an der stationären Ausrüstung angeordnet sind.

8. System nach einem der Ansprüche 1 bis 7, wobei das System zwei oder mehr Behältereinheiten (4) umfasst, die dazu ausgebildet sind, ein biopharmazeutisches Produkt (BP) zu enthalten, wobei im Gebrauch die zwei oder mehr Behältereinheiten übereinander gestapelt werden können, und wobei lokale Gasführungswände (45) an den Ober- und Unterseiten jeder Behältereinheit vorgesehen sind.

9. System nach einem der Ansprüche 1 bis 8, ferner umfassend horizontale Trennplatten (22), die zwischen zwei benachbarten Behältereinheiten (4) angeordnet sind, um eine Gasführung bereitzustellen.

10. System nach einem der Ansprüche 1 bis 9, wobei die stationäre Ausrüstung (1) ein Gebläse (71) und einen Wärmetauscher (72) umfasst und/oder wobei die stationäre Ausrüstung (1) eine elektrische Heizeinrichtung (73) umfasst.

11. System nach einem der Ansprüche 1 bis 10, wobei die Palette eine Grundfläche von im Wesentlichen 80 cm mal 120 cm aufweist und wobei für die Palette ein Palettendichteindex PDI = BPV/PLV definiert ist, wobei BPV das Gesamtvolumen des im Gebrauch in den Behältereinheiten enthaltenen biopharmazeutischen Produkts (BP) ist und wobei PLV die Höhe der Palette multipliziert mit der Grundfläche der Palette, d. h. das Gesamtvolumen, ist, und wobei PDI zwischen 0,1 und 0,3 liegt.

12. System nach Anspruch 3 oder Anspruch 4 oder einem der Ansprüche 5 bis 11, sofern von Anspruch 3 oder Anspruch 4 abhängig, wobei die Schutzverpackung (6) eine lokale innere Gasführungswand (46) umfasst, die in Kontakt mit dem flexiblen Beutel (5) steht, wodurch jeglicher Kontakt zwischen dem zirkulierenden Gas und der Außenfläche (56) des flexiblen Beutels (5) verhindert und ein Wärmefluss dazwischen ermöglicht wird.

13. Palette (2), die dazu ausgebildet ist, in einem System zum Einfrieren, Lagern, Transportieren und Auftauen von biopharmazeutischem Produkt verwendet zu werden, wobei die Palette dazu ausgebildet ist, eine oder mehrere Behältereinheiten (4) zu tragen, die jeweils ein biopharmazeutisches Produkt (BP) enthalten,
wobei die Palette ein erstes Seitenplenum (31) umfasst, das ein oder mehrere Gasförderkanäle darin aufweist und dazu ausgebildet ist, fluidisch mit einem Ausblasauslass einer Gaszirkulationseinheit gekoppelt zu werden,
wobei das erste Seitenplenum (31) eine Vielzahl von Auslassöffnungen (33) aufweist, die hin zu einem Innenvolumen (CV) der Palette gerichtet sind, wobei im Gebrauch Gas im Wesentlichen in einer horizontalen Richtung (XY) in dem Innenvolumen der Palette zirkuliert, wobei die Palette ferner ein zweites Seitenplenum (32) umfasst, das eine Vielzahl von Einlassöffnungen (34) aufweist, wobei die Auslassöffnungen (33) des ersten Seitenplenums dazu ausgebildet sind, Gas auszublasen, während die Einlassöffnungen des zweiten Seitenplenums dazu ausgebildet sind, Gas anzusaugen und das Gas zu der Gaszirkulationseinheit (7) zurückzuführen, wobei die Einlassöffnungen in der horizontalen Richtung (XY) den Auslassöffnungen gegenüberliegend angeordnet sind, wobei die Seitenplenen (31, 32) ferner Stützen zum Abstützen der Behältereinheiten (4) bilden,
**dadurch gekennzeichnet, dass** die Palette ferner eine Rütteleinrichtung (15) umfasst.

## Revendications

1. Système de congélation, stockage, transport et décongélation de produits biopharmaceutiques, le système comprenant un équipement stationnaire (1) et une ou plusieurs palettes mobiles (2), chaque palette étant configurée pour porter une ou plusieurs unités de conteneur (4), contenant chacune un produit biopharmaceutique (BP),
l'équipement stationnaire comprenant une unité de circulation de gaz (7) configurée pour provoquer une circulation de gaz froid et/ou de gaz chaud depuis une entrée d'aspiration (76) vers une sortie de soufflage (77),
dans lequel il est prévu au moins un premier plénum latéral (31 ; 131), comportant en son sein un ou plusieurs canaux d'acheminement de gaz, configuré pour être couplé fluidiquement à la sortie de soufflage de l'unité de circulation de gaz,
le premier plénum latéral (131) comporte une pluralité d'orifices de sortie (33) dirigés vers un volume intérieur (CV) de la palette, dans lequel, en utilisation, le gaz circule sensiblement dans une direction horizontale (XY) dans le volume intérieur de la palette,
**caractérisé en ce que** le système comprend en outre un dispositif d'agitation (15).

2. Système selon la revendication 1, dans lequel il est en outre prévu un second plénum latéral (32 ; 132) comportant une pluralité d'orifices d'entrée (34), dans lequel les orifices de sortie (33) du premier plénum latéral sont adaptés pour souffler le gaz tandis que les orifices d'entrée du second plénum sont adaptés pour aspirer le gaz et renvoyer le gaz vers l'unité de circulation de gaz (7), où les orifices d'entrée sont agencés à l'opposé des orifices de sortie dans la direction horizontale (XY).

3. Système selon l'une quelconque des revendications 1 à 2, dans lequel le système comprend une ou plusieurs unités de conteneur (4) configurées pour contenir un produit biopharmaceutique (BP) et dans lequel les unités de conteneur sont des ensembles de poche, chaque ensemble de poche comprenant une poche souple à usage unique (5) et soit un cadre d'emballage de protection soit un boîtier d'emballage de protection (6), chaque poche souple à usage unique étant configurée pour contenir un produit biopharmaceutique (BP).

4. Système selon la revendication 3, dans lequel, en utilisation du système, les poches souples à usage unique s'étendent sensiblement dans la direction horizontale (XY).

5. Système selon l'une quelconque des revendications 1 à 4, dans lequel il est en outre prévu des turbulateurs (44) qui favorisent un écoulement de gaz turbulent le long des côtés supérieur et inférieur des unités de conteneur.

6. Système selon l'une quelconque des revendications 1 à 5, dans lequel les plénums latéraux (31, 32) sont agencés sur la palette.

7. Système selon l'une quelconque des revendications 1 à 5, dans lequel les plénums latéraux (131, 132) sont agencés sur l'équipement stationnaire.

8. Système selon l'une quelconque des revendications 1 à 7, dans lequel le système comprend deux ou plusieurs unités de conteneur (4) configurées pour contenir un produit biopharmaceutique (BP), dans lequel, en utilisation, les deux ou plusieurs unités de conteneur peuvent être empilées les unes sur les autres, et dans lequel il est prévu des parois locales de guidage de gaz (45) sur les côtés supérieur et inférieur de chaque unité de conteneur.

9. Système selon l'une quelconque des revendications 1 à 8, comprenant en outre des plaques de séparation horizontales (22) agencées entre deux unités de conteneur voisines (4) pour fournir un guidage de gaz.

10. Système selon l'une quelconque des revendications 1 à 9, dans lequel l'équipement stationnaire (1) comprend une soufflante (71) et un échangeur de chaleur (72), et/ou dans lequel l'équipement stationnaire (1) comprend un réchauffeur électrique (73).

11. Système selon l'une quelconque des revendications 1 à 10, dans lequel la palette présente une empreinte au sol d'environ 80 cm sur 120 cm et dans lequel il est défini pour la palette un indice de densité de palette PDI = BPV/PLV, où BPV est le volume total du produit biopharmaceutique (BP) contenu, en utilisation, dans les unités de conteneur et où PLV est la hauteur de la palette multipliée par l'empreinte au sol de la palette, c'est-à-dire le volume global, et dans lequel PDI est compris entre 0,1 et 0,3.

12. Système selon la revendication 3 ou la revendication 4, ou l'une quelconque des revendications 5 à 11 lorsqu'elle dépend de la revendication 3 ou de la revendication 4, dans lequel l'emballage de protection (6) comprend une paroi de guidage interne locale de gaz (46) en contact avec la poche souple (5), empêchant ainsi tout contact entre le gaz circulant et la surface extérieure (56) de la poche souple (5), et permettant un flux thermique entre eux.

13. Palette (2) configurée pour être utilisée dans un système de congélation, stockage, transport et décongélation de produit biopharmaceutique, la palette étant configurée pour porter une ou plusieurs unités de conteneur (4), contenant chacune un produit biopharmaceutique (BP),
dans laquelle la palette comprend un premier plénum latéral (31) comportant en son sein un ou plusieurs canaux d'acheminement de gaz, configuré pour être couplé fluidiquement à une sortie de soufflage d'une unité de circulation de gaz,
le premier plénum latéral (31) comporte une pluralité d'orifices de sortie (33) dirigés vers un volume intérieur (CV) de la palette, dans laquelle, en utilisation, le gaz circule sensiblement dans une direction horizontale (XY) dans le volume intérieur de la palette, la palette comprenant en outre un second plénum latéral (32) comportant une pluralité d'orifices d'entrée (34), dans laquelle les orifices de sortie (33) du premier plénum latéral sont configurés pour souffler le gaz tandis que les orifices d'entrée du second plénum latéral sont configurés pour aspirer le gaz et renvoyer le gaz vers l'unité de circulation de gaz (7), où les orifices d'entrée sont agencés à l'opposé des orifices de sortie dans la direction horizontale (XY), dans laquelle les plénums latéraux (31, 32) forment en outre des montants pour supporter les unités de conteneur (4),
**caractérisée en ce que** la palette comprend en outre un dispositif d'agitation (15).
